Europäisches Patentamt

European Patent Office

Office européen des brevets

(11) Veröffentlichungsnummer: **0 305 407**
**B1**

(12) **EUROPÄISCHE PATENTSCHRIFT**

(45) Veröffentlichungstag der Patentschrift: 05.12.90

(51) Int. Cl.⁵: **A 61 K 31/20**

(21) Anmeldenummer: 87904492.3

(22) Anmeldetag: 07.07.87

(86) Internationale Anmeldenummer:
**PCT/DE87/00307**

(87) Internationale Veröffentlichungsnummer:
**WO 88/00465 28.01.88 Gazette 88/03**

(54) **VERWENDUNG VON DICARBONSÄUREN MIT 7-13 KOHLENSTOFFATOMEN ODER PHYSIOLOGISCH VERTRÄGLICHEN SALZEN DAVON ZUR HERSTELLUNG EINES ARZNEIMITTELS ZUR TOPISCHEN BEHANDLUNG VON ROSAZEA.**

(30) Priorität: 11.07.86 DE 3623862

(43) Veröffentlichungstag der Anmeldung:
08.03.89 Patentblatt 89/10

(45) Bekanntmachung des Hinweises auf die
Patenterteilung:
05.12.90 Patentblatt 90/49

(84) Benannte Vertragsstaaten:
AT BE CH DE FR GB IT LI LU NL SE

(56) Entgegenhaltungen:
US-A-4 386 104

British Journal of Dermatology, Vol. 114, no 4, 1986, P.T. Bladon et al.: "Topical azelaic acid and the treatment of acne: a clinical and laboratory comparison with oral tetracycline", see pages 439-499

The Merck Manual of Diagnosis and Therapy, 14. Edition, 1982, Merck, Sharp & Dohme Research Laboratories (Rahway, N.Y., US), pages 2050-2051, see paragraph "Rosacea"

(73) Patentinhaber: SCHERING AKTIENGESELLSCHAFT Berlin und Bergkamen Müllerstrasse 170/178 Postfach 65 03 11 D-1000 Berlin 65 (DE)

(72) Erfinder: NAZZARO-PORRO, Marcella Via Viminale, 38 I-00184 Roma (IT)

(56) Entgegenhaltungen:
Dorland's Illustrated Medical Dictionary, 26. Edition, 1985, W.B. Saunders Company, (Philadelphia, US), page 1161

British Journal of Dermatology, Band 111, Nr. 1, 1984 A.S. Breathnach: "Azelaic acid", pages 115-120

J. Invest. Dermatol., vol. 84, No. 5, 1985, M. Nazzaro-Porro et al.: "Possible mechanism of action of azelaic acid on acne", see page 451

Pschyrembel, 255. Aufl., 1986, Seite 1469, rechte Spalte

EP 0 305 407 B1

**Beschreibung**

Die Erfindung betrifft die Verwendung von Dicarbonsäuren mit 7 bis 13 Kohlenstoffatomen oder physiologisch verträglichen Salzen davon für die Herstellung eines Arzneimittels zur topischen Behandlung von Rosazea.

Die Verwendung von Dicarbonsäuren mit 7 bis 13 Kohlenstoffatomen zur Behandlung hyperpigmentärer Dermatosen, von Hauthyperpigmentationen oder Akne ist bereits bekannt (U.S.-Patent 4.292.326 und U.S.-Patent 4.386.104).

Rosacea ist eine chronisch hyperämische Krankheit des Gesichts. Sie ist gekennzeichnet durch ein persistierendes Erythem mit akuten Entzündungsphasen unter Ausbildung von Ödemen, Papeln und Pusteln.

Die Ätiologie der Krankheit ist nicht bekannt, und das histologische Bild ist nicht pathognomonisch.

Die erfolgreiche Behandlung der Rosazea bereitet Schwierigkeiten. Während Papeln und Pusteln auf verschiedene topische Mittel und auf systemisch wirkende Antibiotika ansprechen, gibt es keine definitive Behandlung des Erythems.

Es wurde nun gefunden, daß die topische Behandlung mit einer Dicarbonsäure mit 7 bis 13 Kohlenstoffatomen oder einem physiologisch verträglichen Salz davon in einem pharmazeutisch verträglichen Träger einen überraschend günstigen Effekt sowohl auf die entzündliche Komponente (Papeln und Pusteln) als auch auf die vaskulare Komponente der Rosazea (Erythem und Teleangiektasien) hat.

In einer klinischen Studie wurden 12 Patienten mit Rosazea mit einer 20% (Gewichtsprozent)-haltigen Azelainsäurecreme 2 mal täglich 6 Monate lang behandelt. Keiner der Patienten war 3 Monate vor Behandlungsbeginn mit anderen Medikamenten gegen Rosazea behandelt worden.

Die Zahl der Patienten umfaßte 10 Frauen und 2 Männer im Alter zwischen 31 und 76 Jahren (siehe Tabelle).

Hyperämie und Teleangiektasie waren vorherrschend bei den Patienten 3, 4, 7, 10 und 11, die entzündliche Form überwog bei den Patienten 1, 5, 8, 11 und 12. Erytheme an der Nase und fettige Haut waren bei den beiden Männern besonders ausgeprägt. Die Krankheit hatte bereits 1—10 Jahre bestanden und auf andere Therapieversuche nicht oder nur unvollkommen angesprochen. Vor, während und nach der Behandlung wurden Blutproben untersucht und photographische Aufnahmen gemacht.

Der günstige therapeutische Effekt der Azelainsäurecreme manifestierte sich in einer fortschreitenden Abnahme der Zahl der entzündlichen Läsionen und eines Rückgangs des Erythems und der Teleangiektasie.

Nach 2- bis 4monatiger Therapie wurden zufriedenstellende Ergebnisse bei den Patienten 1, 3, 4, 9, 11, 12 und nach 5- bis 8monatiger Therapie bei den Patienten 2, 5, 6, 7, 8 und 10 erhalten. Abgesehen von einer Hautirritation bei einigen Patienten an den ersten Therapietagen wurden keine lokalen oder systemischen Nebenwirkungen beobachtet.

Alle Patienten stehen noch nach Abschluß der Behandlung unter Kontrolle. Es gab einige Rückfälle, insbesondere nach Einwirkung von Sonnenlicht, nach Alkoholgenuß oder psychischem Stress. Nach erneuter Anwendung der Creme konnten alle Rückfälle rasch behoben werden.

Die Herstellung der topischen Zubereitung erfolgt in üblicher Weise, indem man die Wirkstoffe mit geeigneten Zusätzen in die gewünschte Applikationsform, wie zum Beispiel Lösungen, Lotionen, Cremes, Salben, Pasten oder Pflaster, überführt, wobei Lotionen, Cremes und Salben bevorzugt sind. In der so formulierten Zubereitung ist die Wirkstoffkonzentration von der Applikationsform abhängig. Bei Lotionen, Cremes und Salben wird vorzugsweise eine Wirkstoffkonzentration von 5 bis 30 Gewichtsprozent verwendet.

Die Lotionen oder Cremes (Öl/Wasser-Emulsionen) und die Salben (Wasser/Öl-Emulsionen) können in konventioneller Weise unter Verwendung konventioneller Emulgatoren hergestellt werden (Kirk Othmer: Enzyclopedia of Chemical Technology, 3. Auflage, 1979; John Wiley & Sons, New York, Vol. 8, Seiten 900—930, und Dr. Otto-Albrecht Neumüller: Römpps Chemie Lexikon, 7. Auflage, 1973; Franckh'sche Verlagshandlung Stuttgart, Seiten 1009—1013). Die für diese Emulsionen verwendeten Wachse, Emulgatoren und übrigen Zusätze sind die gleichen, wie man sie konventioneller Weise verwendet (Dr. Otto-Albrecht Neumüller: Römpps Chemie Lexikon, 7. Auflage, 1973; Franckh'sche Verlagshandlung Stuttgart, Seiten 1427 und 1428).

Die erfindungsgemäße Verwendung der topischen Zubereitung in Form einer Öl/Wasser-Emulsion kann aus hyxdrophilen und/oder lipophilen Wirkstoffen, Fettphase, Öl/Wasser-Emulgator, wäßriger Phase und Konservierungsmittel bestehen.

Als hydrophile und/oder lipophile Zusätze können Feuchthaltefaktoren (Hydrokomplexe), wie zum Beispiel Propylenglykol, Glycerin, Polyäthylenglykole oder Aminosäuregemische, Puroba-Oil (= Jojoba-Öl), Vitamine (vorzugsweise Vitamin A und E), Vitalkomplexe (wie zum Beispiel Placentaextrakte), Enzyme, Kräuterauszüge (wie zum Beispiel Hammamelis Extrakt oder Kamillenextrakt) oder Proteine (wie zum Beispiel Collagen) eingesetzt werden. Als ölige Phase oder als Fettphase in der Öl/Wasser-Emulsion eignen sich Kohlenwasserstoffe, wie zum Beispiel Squalen, Vaseline, Paraffine oder Stearin, oder Wachse, wie zum Beispiel Bienenwachs. Geeignete Öl/Wasser-Emulgatoren sind beispielsweise Stearylalkohol, Polyoxyäthylenstearate (wie zum Beispiel MYRJ[(R)]), Komplexemulgatoren (wie zum Beispiel Amphoterin[(R)]) und Sorbitanfettsäureester (wie zum Beispiel Tween 80[(R)]) oder Carboxyvinylpolymerisate (wie zum Beispiel Carbopol[(R)]. Die wäßrige Phase kann zusätzlich noch Puffersubstanzen, wie zum Beispiel das Dinatriumsalt der Äthylendiamin-N,N,N',N'-tetraessigsäure und Konservierungsmittel, wie Benzoesäure, Chlorquinaldol, Parabene oder Benzalkoniumchlorid, enthalten.

In der Öl/Wasser-Emulsion beträgt der Anteil der inneren Emulsion vorzugsweise 10 bis 49 Gewichtsprozent, die Teilchengröße der inneren Emulsion liegt vorzugsweise zwischen 1 µ und 100 µ.

Die erfindungsgemäße Verwendung der topischen Zubereitung in Form einer Wasser/Öl-Emulsion besteht ebenfalls aus hydrophilen und/oder lipophilen Zusätzen, wie sie auch in der Öl/Wasser-Emulsion verwendet werden, Fettphase, Wasser/Öl-Emulgator und wäßriger Phase. Als ölige Phase oder Fettphase der Wasser/Öl-Emulsion können Kohlenwasserstoffe, zum Beispiel Paraffine und Vaseline, synthetische, pflanzliche und tierische Öle bzw. Wachse (wie zum Beispiel Olivenöl, Erdnußöl, feines Knochenöl, Mandelöl, Lanolin, Bienenwachs oder Sonnenblumenöl) eingesetzt werden, als wäßrige Phase gereinigtes demineralisiertes Wasser und also Wasser/Öl-Emulgator Wollfett (= Lanolin), Fettalkohole, wie zum Beispiel Cetylalkohol, Myristylalkohol, Stearylalkohol oder Cerylalkohol, Fettsäureester, wie zum Beispiel Bienenwachs (Cera alba) oder Wachsalkoholester oder Michester (wie zum Beispiel Dehymuls[(R)]).

In der Wasser/Öl-Emulsion beträgt der Anteil der inneren Emulsion vorzugsweise 30 bis 49 Gewichtsprozent, die Teilchengröße der inneren Emulsion liegt vorzugsweise zwischen 1 µ und 100 µ.

Das feindisperse System wird zusätzlich mit dem mikronisierten Wirkstoff (Korngröße vorzugsweise 1 bis 20 µ) und gegebenenfalls noch mit Duftstoffen, wie zum Beispiel diejenige der Crematest[(R)]-Serie, versetzt und bis zur gleichmäßigen Verteilung derselben gerührt.

Dem Arzneimittel können keratolytische Mittel, wie zum Beispiel Salicylsäure, Vitamin-A-Säure, Resorcin, Phenol, Cresol und dergleichen, zugesetzt werden.

Als Wirkstoffe werden Dicarbonsäuren mit 7 bis 13 Kohlenstoffatomen oder physiologisch verträgliche Salze der Dicarbonsäuren eingesetzt.

Zu den erfindungsgemäß verwendeten Dicarbonsäuren gehören insbesondere Pimelinsäure, Suberinsäure, Azelainsäure, Sebazinsäure, 1,9-Nonandicarbonsäure, 1,10-Decandicarbonsäure und 1,11-Undecandicarbonsäure.

Zu den physiologisch verträglichen Salzen zählen Alkalimetallsalze, wie Natrium- and Kaliumsalze, ferner Salze mit basischen Aminoverbindungen und organischen Aminen, wie zum Beispiel Arginin, Lysin oder N-Methylglucamin.

In einer bevorzugten Ausführungsform wird Azelainsäure als Dicarbonsäure verwendet.

## Beispiel 1

|  | Gewichtsprozent |
|---|---|
| Azelainsäure | 20,0 |
| Benzoesäure | 0,1 |
| Salicylsäure | 2,0 |
| Glycerinmonostearat | 2,0 |
| Cetylalkohol | 3,0 |
| Polyoxyethylen(20)sorbitanmonooleat | 5,0 |
| Natrium-laurylethersulfat | 10,0 |
| Ethanolamin-laurylethersulfat | 1,0 |
| Olivenöl | 2,0 |
| Ascorbinsäure | 1,0 |

mit destilliertem Wasser auffüllen auf 100,0 Gewichtsprozent.

Azelainsäure, Benzoesäure, Polyoxyethylen(20)sorbitanmonooleat, Natrium-laurylethersulfat, Ethanolamin-laurylethersulfat und Wasser werden auf 60°C erwärmt und mit der Mischung von Salicylsäure, Glycerinmonostearat, Cetylalkohol, Olivenöl und Ascorbinsäure bei 40°C dispergiert.

# EP 0 305 407 B1

### Beispiel 2

| | Gewichtsprozent |
|---|---|
| Azelainsäure-Kaliumsalz (70% neutralisiert) | 20,0 |
| Propylenglykol | 15,0 |
| Squalen | 10,0 |
| Cetylalkohol | 3,0 |
| Vaseline | 4,0 |
| Bienenwachs | 2,0 |
| Polyoxyethylen(20)sorbitanmonooleat | 5,0 |

mit destilliertem Wasser auffüllen auf 100,0 Gewichtsprozent.

Propylenglykol und Wasser werden auf 60°C erwärmt. Squalen, Cetylalkohol, Vaseline und Bienenwachs werden bei 60—80°C geschmolzen. Die Wasserphase wird unter Rühren zur Fettphase gegeben. Anschließend wird unter Rühren auf Raumtemperatur abgekühlt und 20 Gewichtsprozent (20 g auf 80 g Grundlage) Azelainsäure (mikronisiert) eingearbeitet und homogenisiert.

| Patienten-Nr. | Tag der Untersuchung | Patienten-Initialen | Alter (Jahre) | Geschlecht | Dauer der Krankheit vor Behandlungsbeginn (Jahre) |
|---|---|---|---|---|---|
| 1 | 07/06/83 | F.M. | 56 | F | 8 |
| 2 | 26/06/84 | Z.R. | 35 | F | 1 |
| 3 | 07/07/84 | T.E. | 31 | F | 9 |
| 4 | 29/08/84 | G.A. | 43 | F | 4 |
| 5 | 16/10/84 | B.L. | 46 | F | 3 |
| 6 | 04/03/85 | B.C. | 58 | M | 1 |
| 7 | 05/03/85 | D.A. | 34 | F | 4 |
| 8 | 06/03/85 | M.M. | 45 | F | 10 |
| 9 | 21/03/85 | V.M. | 76 | F | 7 |
| 10 | 15/05/85 | T.R. | 60 | M | 1 |
| 11 | 29/05/85 | M.M. | 71 | F | 10 |
| 12 | 29/01/86 | R.L. | 65 | F | 8 |

## Patentansprüche

1. Verwendung von Dicarbonsäuren mit 7 bis 13 Kohlenstoff atomen oder physiologisch verträglichen Salzen davon für die Herstellung eines Arzneimittels zur topischen Behandlung von Rosazea.

2. Verwendung nach Anspruch 1, dadurch gekennzeichnet, daß das Arzneimittel in Form einer Lotion, Creme oder Salbe vorliegt.

3. Verwendung von Azelainsäure als Dicarbonsäure nach Anspruch 1.

## Revendications

1. Application d'acides dicarboxyliques contenant de 7 à 13 atomes de carbone, ou de sels physiologiquement acceptables d'acides de genre, à la préparation d'un médicament destiné au traitement topique de l'acné rosacée.

2. Application selon la revendication 1 caractérisée en ce que le médicament est sous la forme d'une lotion, d'une crème ou d'une pommade.

3. Application de l'acide azélaïque comme acide dicarboxylique selon la revendication 1.

**Claims**

1. Use of dicarboxylic acids having from 7 to 13 carbon atoms or of physiologically tolerable salts thereof for the preparation of a medicament for the topical treatment of rosacea.

2. Use according to claim 1, characterized in that the medicament is in the form of a lotion, cream or ointment.

3. Use of azelaic acid as dicarboxylic acid according to claim 1.